# FASCICULE DE BREVET EUROPEEN

(11) **EP 0 366 534 B1**
(45) Date de publication et mention de la délivrance du brevet: **31.03.1993**
(21) Numéro de dépôt: 89402916.4
(22) Date de dépôt: 24.10.1989
(51) Int. Cl.: B65D 51/24, B65D 51/28

(54) **Conditionnement destiné à modifier la composition d'un liquide**
Zum Ändern der Zusammensetzung einer Flüssigkeit bestimmte Verpackung
Package to change the composition of a liquid

(30) Priorité: 28.10.1988 FR 8814211
(43) Date de publication de la demande: 02.05.1990
(73) Titulaire: TRANSPHYTO SA, 63100 Clermont-Ferrand (FR)
(72) Inventeur: Lontrade, Jean-Pierre, F-63000 Clermont Ferrand (FR); Chibret, Henri, F-63000 Clermont Ferrand (FR); Schwadrohn, Gérard, F-06000 Nice (FR)
(74) Mandataire: Thibon-Littaye, Annick

(56) Documents cités:
- EP-A- 0 077 556
- EP-A- 0 236 033
- EP-A- 0 357 288
- FR-A- 1 367 129

## Description

La présente invention concerne l'industrie du conditionnement et de l'épuration des liquides, plus particulièrement dans des récipients à obturateur incorporé et notamment la modification physique ou chimique d'un liquide.

L'invention trouve une application particulièrement intéressante dans le domaine de l'industrie pharmaceutique, notamment pour les produits thérapeutiques d'application locale utilisés en ophtalmologie, dermatologie, gynécologie ainsi qu'en cosmétologie. Elle est bien entendu également applicable par exemple dans le domaine des flacons de produits ménagers ou de produits chimiques.

On connaît des récipients, notamment en matière plastique à parois élastiquement déformables, dont le goulot renferme un obturateur inséparable, mobile à volonté entre une position de fermeture et une position d'ouverture du récipient, du type des robinets à boisseau ou à tiroir.

On a parfois à modifier le liquide distribué, notamment par extraction d'une substance indésirable par passage à travers un élément filtrant ou épurateur extérieur au récipient, ou encore par addition extemporanée d'une substance soluble dans le liquide contenu dans le récipient, ce qui est assez courant pour les solutions thérapeutiques de lyophilisats, et généralement réalisé par confinement de la substance additionnelle dans une enveloppe frangible à l'intérieur du récipient jusqu'au moment de l'utilisation.

Les réponses apportées jusqu'ici à ces problèmes de conditionnement présentent des inconvénients pratiques découlant notamment de la complexité des organes à réunir et, d'autre part, de la fragilité des éléments extérieurs au récipient, ou au contact du liquide avant l'utilisation. De plus, il est nécessaire de concevoir un conditionnement particulier pour chaque application, ce qui limite les séries de fabrication et influe sur les prix de revient industriels.

L'invention a pour but un conditionnement pour liquides, aisément standardisable, permettant de conserver le liquide isolé de l'extérieur et d'éventuelles substances additionnelles, ou d'éléments filtrants ou épurateurs, jusqu'au moment de l'utilisation, composé d'éléments simples à fabriquer et à assembler pour constituer un conditionnement unitaire de manipulation facile, robuste et peu onéreux.

L'invention a également pour but l'épuration extemporanée de petites quantités fractionnaires prélevées d'un liquide à partir d'un contenant au fur et à mesure de son utilisation.

Pour la conservation de tels produits pendant leur stockage, notamment pour des produits destinés à une application thérapeutique, il est souvent nécessaire, voire légalement obligatoire, d'ajouter des conservateurs dissous dans le liquide stocké. Mais ces conservateurs ont le plus souvent des effets indésirables après ou lors de l'application thérapeutique. On constate ainsi souvent des irritations inhibant l'action de produits cicatrisants ou autres.

On se trouve ainsi confronté à un problème apparemment difficile à résoudre : conserver une solution thérapeutique stérile pendant une durée convenable de stockage impliquant l'addition d'un conservateur, et appliquer cette même solution stérile, pure, donc sans conservateur. Ce problème se complique encore du fait que, dans la plupart des applications thérapeutiques locales, l'utilisation d'un conditionnement est fractionnée et, qu'une fois le récipient ouvert, le liquide s'il ne contient pas de conservateur est en contact avec l'air ambiant et perd sa stérilité.

Une solution courante actuelle à ce problème consiste à conditionner les liquides stériles dans des récipients correspondant chacun à l'ensemble d'un traitement que l'on épure éventuellement en totalité avant application, mais au risque d'une perte de stérilité. On a aussi proposé de protéger la stérilité du contenu d'un récipient entre des prélèvements fractionnaires successifs en disposant un filtre stérilisant à la sortie du récipient.

Un tel filtre protège bien la stérilité contre une pollution extérieure du liquide non distribué qui reste dans le récipient, mais laisse subsister le conservateur éventuel dans le liquide distribué avec tous ses inconvénients. Une épuration éventuelle (globale) de la fraction de liquide distribué à la sortie du récipient entraîne un risque de perte de stérilité du liquide appliqué, et des manipulations peu pratiques pour l'utilisateur occasionnel qu'est un patient.

L'invention a pour but de résoudre un tel problème et de permettre de conserver une quantité fractionnable de liquide renfermant une substance indésirable à l'emploi, notamment un conservateur de liquide thérapeutique, et de distribuer ce liquide, à volonté par doses successives, à l'état stérile et épuré de la substance indésirable, sans manipulation particulière.

Selon sa caractéristique principale, l'invention a pour objet un conditionnement pour liquide à épurer, comprenant un récipient dont un goulot renferme un sas de réception d'au moins un élément de traitement du liquide lors de son expulsion hors du récipient, caractérisé en ce que ledit sas est solidaire d'un obturateur ou bouchon mobile déplaçable au moins lors d'une première utilisation entre une position de stockage où il isole l'intérieur du récipient du sas et une position d'utilisation où il permet une communication entre l'intérieur du récipient et l'intérieur du sas, ledit obturateur restant solidaire dudit sas qui présente un volume intérieur constant, et ledit sas étant limité par un élément amont de filtration et de retenue permanente dudit élément de traitement dans le sas.

Le sas renferme avantageusement une substance épuratrice insoluble dans le liquide retenue entre l'élément de filtration amont et un élément de filtration aval.

La construction mécanique et la structure du conditionnement lui permettent de recevoir dans le sas un élément de traitement ou élément de modification du liquide qui ne soit en contact avec le liquide qu'au moment de l'expulsion de ce dernier.

On réalise ainsi un ensemble sas/obturateur qui permet, lors du stockage du récipient avant son utilisation, qu'un élément de modification contenu dans le sas soit complètement isolé du liquide contenu dans le récipient, le récipient pouvant ainsi être stocké dans n'importe quelle position sans que cela nuise à la conservation du produit liquide qu'il contient.

De plus, le fait que le sas soit limité par un filtre à chacune de ses extrémités accroît considérablement les modes de traitement possibles du liquide lors de son utilisation et permet que ce type de conditionnement puisse être utilisé dans de nombreuses applications différentes sans qu'il soit nécessaire de modifier sa structure.

On peut ainsi avantageusement prévoir que le sas renferme une substance épuratrice ou élément de modification du liquide, qu'un élément filtrant amont ou premier élément filtrant contribue à retenir un premier type de particules avant que le liquide n'accède à la substance épuratrice et qu'un élément filtrant aval ou second élément filtrant retienne un second type de particules avant l'expulsion du liquide vers l'extérieur, les deux éléments filtrants jouant en outre le rôle de retenir la substance épuratrice dans le sas.

De plus, le fait que l'obturateur reste solidaire du sas quelle que soit la position de l'ensemble permet, comme on le verra par la suite, qu'en conservant une structure identique pour le conditionnement, celui-ci puisse être utilisé pour dans des applications où l'on désire isoler l'intérieur du sas de l'intérieur du récipient après chaque période d'utilisation et/ou pour des applications où l'on souhaite pouvoir mélanger un produit à l'ensemble du liquide avant une première utilisation.

En outre, un tel conditionnement permet, par sa structure que le volume intérieur du sas soit constant quelle que soit la position de stockage ou d'utilisation du conditionnement, ce qui entraîne que le volume utile du sas soit identique à son volume intérieur, procurant une diminution de l'encombrement global de l'ensemble.

Le sas peut avantageusement renfermer une substance épuratrice insoluble dans le liquide. L'éléments de filtration aval, peut avantageusement être un filtre bactériologique.

Dans une application à une solution thérapeutique dont la substance indésirable, seulement au moment de l'emploi, est un produit d'application locale du groupe comprenant les collyres, les solutions nasales, les lotions dermatologiques et d'une façon générale les produits à appliquer au contact des muqueuses ou à leur périphérie, le sas renferme avantageusement une substance propre à éliminer un conservateur contenu dans le liquide à épurer.

La perméabilité des éléments filtrants, et notamment de l'élément filtrant amont est avantageusement telle qu'une différence de pression minimum prédéterminée (exemple : pression manuelle sur les parois d'un récipient souple) soit nécessaire à la traversée du liquide, afin d'éviter le contact en cas de secousses inopinées entre les utilisations.

Le conditionnement selon l'invention comporte avantageusement des moyens de variation du volume intérieur du récipient, ce qui permet par exemple, par action sur des parois élastiquement déformables, l'expulsion du liquide qu'il contient.

Dans un mode de réalisation particulièrement avantageux, les moyens de variation du volume intérieur du récipient comportent au moins une paroi mobile du récipient, par exemple le fond à la manière d'une seringue de façon à diminuer le volume intérieur du récipient au fur et à mesure de l'expulsion du liquide qu'il contient. Ceci permet de préserver le liquide d'une éventuelle pollution par l'air lors de périodes d'utilisations successives, dans la mesure où l'entrée d'air dans le récipient est évitée par le fait que ce dernier ne remplace pas le liquide expulsé par de l'air.

Dans un mode de réalisation avantageux, le sas et le bouchon forment un ensemble unitaire comprenant un corps tubulaire ; le corps comportant au moins un conduit de passage du liquide à travers le bouchon et un fond qui ferme ces moyens de passage par coopération étanche notamment par ajutage étanche avec le goulot du récipient avant la première utilisation.

L'ensemble sas/bouchon peut coulisser dans le goulot du récipient et peut être amené, à volonté, de la position de stockage où il est isolé de l'intérieur du récipient qu'il bouche, à la position d'utilisation où il met en communication, à travers lui, l'intérieur du récipient avec l'extérieur pour en assurer le remplissage en liquide ou sa distribution.

Pratiquement, l'ensemble sas/bouchon est avantageusement constitué par un corps tubulaire de révolution présentant à son extrémité appelée à communiquer avec l'extérieur, un évasement interne dans une collerette terminale dont la face radiale externe forme un appui annulaire étanche pour la périphérie dudit élément de filtration aval, et il comporte avantageusement un embout distributeur présentant un épaulement annulaire interne appliquant la périphérie de l'élément de filtration aval contre l'épaulement d'appui annulaire de la collerette et présentant des moyens de passage du liquide entre la face externe de l'élément de filtration aval et un canal axial de distribution de l'embout.

Dans un tel conditionnement, une bonne fermeture du récipient en position de stockage et un bon dégagement des passages du liquide en position d'utilisation sont favorisés si l'ensemble sas/bouchon présente une longueur supérieure à celle du goulot du récipient dans lequel il coulisse, le bouchon et le tronçon du corps tubulaire de même diamètre présentant des lèvres ou bourrelets annulaires d'étanchéité dans le goulot en position de stockage et en position d'utilisation ; la collerette formant butée sur le bord du goulot du récipient pour limiter l'enfoncement de l'ensemble unitaire sas/bouchon vers la position d'utilisation où au moins une ouverture latérale du bouchon est dégagée du goulot alors qu'elle est enfermée et isolée de façon étanche dans le goulot en position de stockage.

L'isolement de l'extérieur de l'ensemble sas/bouchon en position de stockage, ainsi qu'en position d'utilisation en dehors des périodes d'utilisation, en même temps que la manoeuvre de mise en position d'utilisation sont avantageusement obtenus avec un capuchon vissant à l'extérieur du goulot et comportant un téton axial d'obturation du canal de distribution de l'embout ainsi qu'un prolongement annulaire interne d'appui contre la face externe de l'embout pour enfoncer manuellement celui-ci dans le goulot vers la position d'utilisation, le capuchon comportant une bague frangible de sécurité prolongeant sa jupe et portant contre le col du récipient de sorte qu'après la première utilisation, le capuchon et l'embout deviennent inopérants à provoquer le déplacement de l'ensemble sas/bouchon.

Selon un mode de réalisation préféré de l'invention, l'ensemble sas/bouchon est avantageusement constitué d'une seule pièce par ledit corps tubulaire et ce dernier comporte, dans son extrémité appelée à communiquer avec l'intérieur du récipient, une chambre de réception d'un produit destiné à être mélangé au liquide lors d'une première mise en position d'utilisation et débouchant par une première ouverture latérale dans la périphérie dudit bouchon et par une seconde ouverture dans ledit sas en regard dudit élément de filtration amont, ladite chambre de réception étant avantageusement dans la position de stockage, isolée de l'intérieur du récipient, et en communication avec ce dernier dans la position d'utilisation, ledit produit se trouvant, lors d'une première mise en position d'utilisation, déversé dans ledit récipient.

Cette structure permet de manière particulièrement avantageuse de combiner l'emploi d'un élément de traitement ou de modification, non soluble dans le liquide, contenu dans le sas et purifiant une dose de liquide à chaque utilisation, avec l'emploi d'un produit soluble dans le liquide, notamment un lyophilisat, contenu dans la chambre ménagée dans le bouchon et qui est déversé dans le récipient lors d'une première mise en position d'utilisation.

Le corps tubulaire comporte avantageusement un épaulement d'appui annulaire étanche dudit élément de filtration amont en périphérie qui constitue la séparation entre le sas et le bouchon. On peut ainsi, sans changer l'encombrement total de l'ensemble sas/bouchon, modifier en fonction de l'application souhaitée et des éléments de modification à mettre en oeuvre, les volumes respectifs du sas et de la chambre par un positionnement longitudinal différent de l'épaulement annulaire d'appui du premier élément de filtration.

De plus, le fait de prévoir l'ensemble sas/bouchon d'une seule pièce, permet d'appliquer aisément l'invention à des conditionnements fabriqués, remplis et fermés sans discontinuité, par exemple selon une méthode de conditionnement extemporané stérile connu sous le nom commercial "BOTTLE-PACK".

Selon un autre mode de réalisation de l'invention, l'ensemble sas/bouchon peut être pratiquement constitué par un corps tubulaire de révolution présentant à son extrémité, appelée à communiquer avec l'intérieur du récipient, un épaulement interne d'appui annulaire d'un premier élément de filtration en périphérie et des moyens d'encliquetage étanches pour un bouchon de même diamètre extérieur que le corps tubulaire dont un bord périphérique applique le premier élément de filtration contre l'épaulement d'appui du corps tubulaire et qui est entaillé de canaux radiaux pour le libre passage du liquide, débouchant en-dessous du premier élément de filtration, côté récipient.

Pour assurer un bon soutien des éléments de filtration et une bonne circulation du liquide, il est avantageux que les faces du bouchon et de l'embout en regard des éléments de filtration, hormis la périphérie, présentent des aspérités d'appui pour les faces des éléments de filtration, qui ménagent entre elles des canaux intercommuniquants de circulation du liquide.

Selon un autre mode de réalisation de l'invention où l'on désire isoler l'intérieur du sas de l'intérieur du récipient entre les périodes d'utilisation afin de préserver le liquide quelle que soit la position dans laquelle est stocké le récipient, l'ensemble sas/obturateur est avantageusement déplaçable à volonté entre ladite position de stockage et ladite position d'utilisation.

A cet effet, on peut avantageusement prévoir que l'embout comporte des moyens de préhension par l'utilisateur permettant d'imprimer à l'ensemble sas/bouchon un mouvement de translation.

Selon une variante de réalisation, l'ensemble sas/bouchon est avantageusement associé à une fermeture par rotation dans un goulot inversé du récipient, le bouchon présentant un fond ajouré et un téton axial encliqueté en rotation dans une paroi obturant partiellement l'extrémité interne du goulot inversé.

Pour des applications dans lesquelles le liquide du récipient est constitué par une émulsion, et notamment si le produit actif à appliquer n'est qu'un composé huileux de cette émulsion, ce qui peut être le cas par exemple lorsque les conditions de conservation d'un composé huileux nécessite qu'il soit mélangé à un composé aqueux, il est particulièrement avantageux que l'élément de filtration amont soit perméable à tous les constituants du liquide et que l'élément de filtration aval soit sélectivement perméable au composé huileux en retenant le composé aqueux dans le sas.

A l'inverse si l'on souhaite retenir le composé huileux, il est avantageux que l'élément de filtration amont soit sélectivement perméable au composé aqueux ou perméable à tous les constituants du liquide et que l'élément de filtration aval soit sélectivement perméable au composé aqueux uniquement, le conservateur éventuellement contenu dans le composé aqueux se trouvant piégé dans le sas.

On décrira maintenant plus en détail une forme de réalisation particulière de l'invention qui en fera mieux comprendre les caractéristiques essentielles et les avantages, étant entendu toutefois que cette forme de réalisation est choisie à titre d'exemple et qu'elle n'est nullement limitative. Sa description est illustrée par les dessins annexés, dans lesquels :
- la figure 1 est une vue schématique, en élévation et en coupe axiale de l'ensemble sas/bouchon d'un conditionnement suivant l'invention inséré dans le goulot d'un récipient, et coiffé d'un capuchon, en position de stockage ;
- la figure 2 est une vue analogue à celle de la figure 1, du même ensemble sas/bouchon en position d'utilisation ;
- la figure 3 est une vue schématique partielle, en perspective, et en coupe axiale de l'assemblage corps/bouchon de l'ensemble sas/bouchon des figures 1 et 2 ;
- la figure 4 est une vue schématique partielle en perspective et en coupe axiale du bouchon de l'assemblage corps/bouchon de la figure 3 ;
- la figure 5 est une vue schématique partielle en perspective et en coupe axiale de l'embout distributeur de l'ensemble sas/bouchon des figures 1 et 2 ;
- la figure 6 est une suite synoptique de schémas montrant la configuration du conditionnement des figures 1 à 5 dans les diverses phases depuis le stockage jusqu'à la distribution du liquide ;
- les figures 8 et 9 sont des vues schématiques en élévation et en coupe axiale de deux variantes de réalisation permettant de rendre le sas isolé de l'intérieur du récipient entre chaque période d'utilisation ;
- et la figure 10 est une vue schématique en élévation et en coupe axiale d'une forme de réalisation de l'ensemble sas/bouchon permettant le déversement d'un produit dans le récipient avant une première utilisation.

Le conditionnement réprésenté sur ces dessins comprend essentiellement un récipient 1, en l'occurence un flacon en polyéthylène à parois élastiquement déformables, dans le goulot 2 duquel est inséré à coulissement axial un ensemble unitaire constitué par un sas 4 terminé du côté du récipient 1 par un bouchon 3 et à l'opposé par un embout distributeur 18. Un capuchon 26 coiffant l'ensemble est vissé à l'extérieur du goulot 2.

Le sas 4 est constitué par un corps tubulaire 7 de révolution dont la base crénelée 31 présente intérieurement un épaulement annulaire 10 d'appui périphérique pour un premier élément de filtration ou élément de filtration amont 8, éventuellement constitué par une membrane perforée, une toile ou une plaque frittée, ainsi qu'une rainure d'encliquetage 11a pour le bouchon 3.

Le bouchon 3 a le même diamètre extérieur que le corps tubulaire 7 qu'il prolonge. Sa face terminale est pleine tandis que sa face opposée, en vis-à-vis du premier élément de filtration 8, présente, en regard de l'épaulement 10 du corps 7, un bord périphérique annulaire 12 entaillé de canaux radiaux 13 débouchant extérieurement face aux crénelures 31 du corps 7 et intérieurement dans une face axiale en retrait hérissée de villosités 22 ménageant entre elles des canaux intercommuniquants 24 assurant un libre passage du liquide en-dessous du premier élément de filtration depuis, ou vers les ouvertures radiales 13. Le bord 12 crénelé du bouchon a un diamètre extérieur un peu plus petit que le diamètre intérieur des crénelures 31, pour former un canal collecteur périphérique 34 des ouvertures 13.

Le premier élément de filtration 8 est ainsi maintenu par sa périphérie, appliqué de façon étanche contre l'épaulement 10 du corps 7, par le bord périphérique 12 du bouchon 3 encliqueté par une nervure périphérique latérale 11b dans la rainure 11a en bout du corps 7. De plus, il est maintenu sur tout le reste de sa surface, côté récipient 1, par les villosités 22 du bouchon 3.

A son extrémité opposée au bouchon 3, le corps 7 se termine intérieurement par un évasement 14 et extérieurement par une collerette 15 dont la face radiale externe 16 forme un appui annulaire étanche pour la périphérie d'un second élément de filtration ou élément de filtration aval 5, éventuellement constitué par une membrane microporeuse, un filtre bactériologique. La face latérale externe de la collerette 15 présente une rainure circulaire 17a qui coopère avec une nervure périphérique interne 17b de la pipe de l'embout distributeur 18 pour en assurer l'encliquetage.

L'embout 18 présente un canal axial 21 de distribution débouchant, d'une part, extérieurement et, d'autre part, au centre d'une face hérissée de villosités 23 ménageant entre elles des canaux intercommuniquants 20 et bordée par un épaulement annulaire périphérique 19 formant un appui annulaire étanche pour la périphérie du second élément de filtration 5 en regard de la face radiale externe 16 du corps 7.

Le second élément de filtration 5 est ainsi maintenu, par sa périphérie, de façon étanche entre le corps 7 et l'embout 18 encliquetés l'un sur l'autre. Il est de plus soutenu par les villosités 23 sur tout le reste de sa surface et peut être librement traversé par le liquide depuis, ou vers, le canal de distribution 21.

Entre le premier élément de filtration 8, le second élément de filtration 5 et la paroi interne du corps 7 est délimitée une enceinte formant un sas 4 que l'on peut remplir éventuellement partiellement ou complètement d'une substance soluble ou non, modificatrice du liquide 32 à distribuer. Une telle substance peut être éventuellement un sel ou un lyophilisat ou une substance épuratrice.

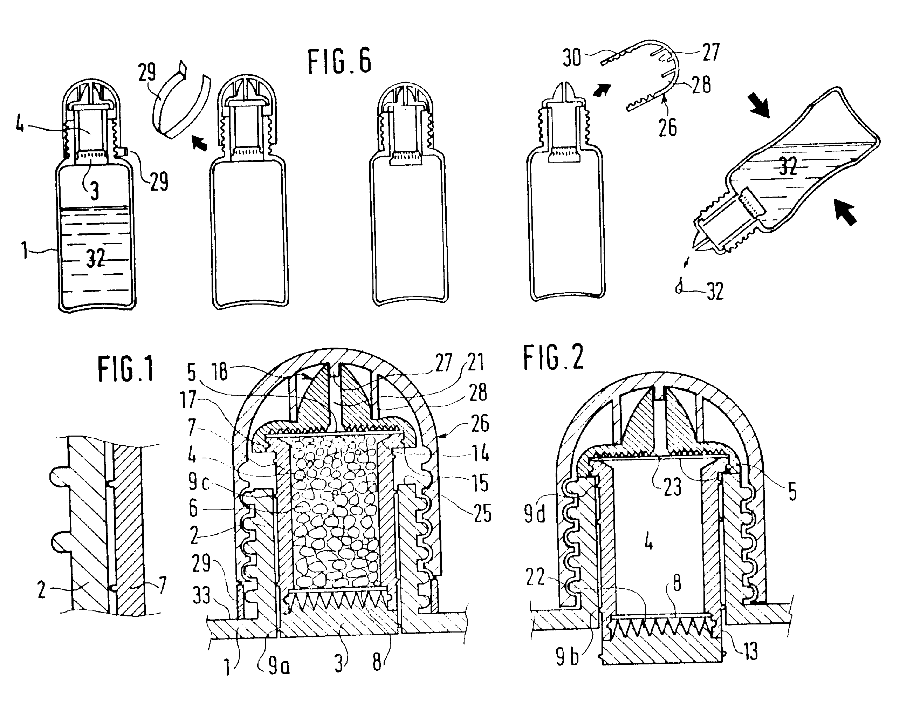

L'étanchéité de l'ensemble sas 4/bouchon 3 dans le goulot est assurée à chaque extrémité du goulot 2 en position de stockage (figure 1) par des nervures annulaires périphériques 9a du bouchon 3 et 9c du corps 7 et en position d'utilisation (figure 2) par des nervures annulaires périphériques 9b et 9d du corps 7.

La longueur de l'ensemble sas 4/bouchon 3 est telle que, en position de stockage (figure 1), la base du bouchon 3 ne dépasse pratiquement pas le bord intérieur du goulot 2 dans le récipient 1 alors qu'en position d'utilisation (figure 2) le bouchon 3 pénètre à l'intérieur du récipient 1 suffisamment pour que les canaux 13 débouchent librement en-dessous du bord intérieur du goulot 2 alors que le bord radial inférieur de la collerette 15 est en butée sur le bord extérieur 25 du goulot 2 du récipient.

Dans une position d'utilisation (figure 2) le bord périphérique de l'embout 18 porte, sans solution de continuité, contre le bord extérieur 25 du goulot du récipient, ce qui ne donne aucune prise pour ramener inopportunément l'ensemble sas/bouchon en position de stockage (figure 1). En variante, le bord de l'embout pourrait s'encastrer dans un évidement annulaire du bord du goulot.

Le capuchon 26 est vissé sur le bord extérieur fileté du goulot 2 du récipient 1. Sa jupe taraudée est prolongée par une bague de garantie frangible 29, aisément arrachable pour la première utilisation du conditionnement. Cette bague 29 bute normalement pendant le stockage sur le col 33 du récipient (figure 1) pour interdire tout vissage inopportun vers la position d'utilisation (figure 2). Un téton axial 27 obture le canal de distribution 21 de l'embout 18 tandis qu'un prolongement annulaire interne 28 appuie sur la face externe de l'embout 18. Lorsque, après avoir arraché la bague de garantie 29, on visse manuellement le capuchon 26, celui-ci enfonce l'ensemble embout 18/sas 4/bouchon 3 jusqu'à la butée de la collerette 15 sur le bord 25 du goulot, qui détermine la position d'utilisation (figure 2), le canal de distribution 21 restant obturé par le téton 27. Il est alors possible de dévisser et retirer le capuchon 26 du conditionnement, sans entraîner l'ensemble embout 18/sas 4/bouchon 3.

On peut alors distribuer le liquide 32 contenu dans le récipient 1 en pressant les parois pour forcer le liquide à travers le sas 4 et ses éléments filtrants 8 et 5 et le distribuer par le canal 21 de l'embout 18.

En cas de distributions fractionnées du liquide, on remet en place le capuchon 26 qui isole le sas 4 et par suite l'intérieur du récipient 1 de l'extérieur. L'air aspiré par le rappel élastique des parois du récipient 1 est filtré par les éléments filtrants 5 et 8, éventuellement par la substance 6 enfermée dans le sas et protège le liquide 32 restant de toute pollution, aussi bien que l'intérieur du sas.

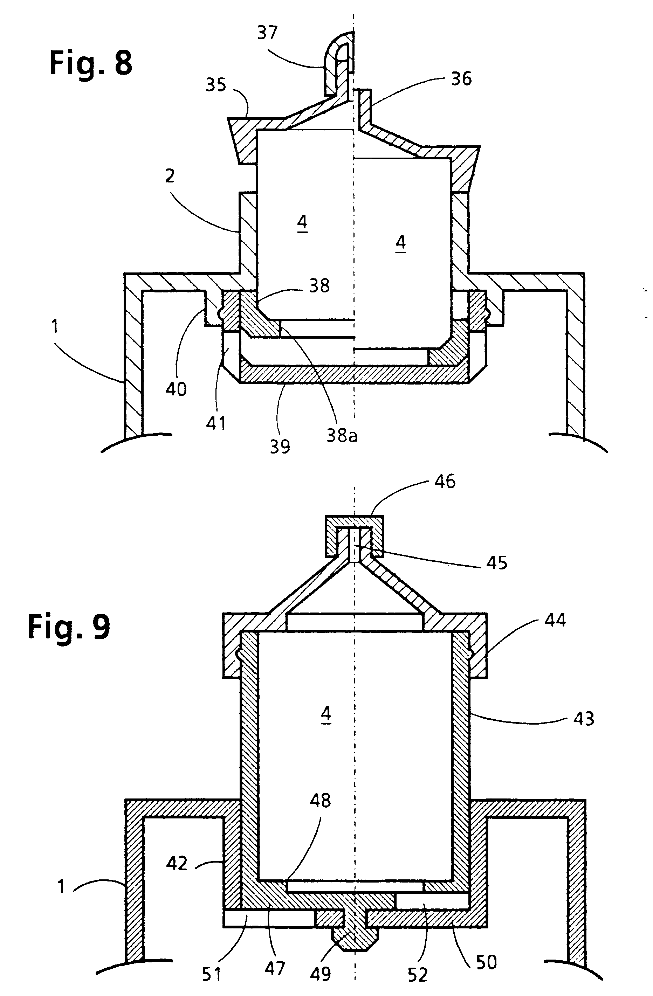

Les variantes de réalisation représentées aux figures 8 et 9 concernent un conditionnement plus particulièrement destiné à des applications pour lesquelles on souhaite qu'entre différentes périodes d'utilisation, l'intérieur du sas 4 soit isolé de l'intérieur du récipient 1, quelle que soit la position dans laquelle est stocké le conditionnement.

Les représentations de ces variantes sont schématiques et les éléments filtrants n'y ont pas été représentés pour des raisons de clarté.

Le conditionnement de la figure 8 comprend un sas 4 associé à une fermeture à translation axiale dans le goulot 2 lisse d'un récipient 1. Le sas 4 est coiffé, à son extrémité externe, par un embout cônique 35 soudé ou collé ou vissé terminé par un ajutage axial 36 obturable à volonté par un capuchon classique 37 et constituant des moyens de préhension permettant à un utilisateur d'imprimer un mouvement de translation à l'ensemble sas 4/bouchon 38. L'extrémité interne du sas 4 constituée par le bouchon 38 coulisse dans un embout borgne 39 encliqueté dans un rebord annulaire interne 40 issu du col du récipient 1 et présentant des ouvertures latérales 41 au voisinage du fond borgne. Le bouchon 38 présente à son extrémité externe un rebord annulaire emboîtant 38a extérieurement tronconique, cependant que le fond borgne de l'embout 39 présente intérieurement une conicité complémentaire de celle du rebord 38a du bouchon 38. Les dimensions respectives du bouchon 38 dans l'embout borgne 39 et leur débattement sont tels que, lorsque l'ensemble embout 35/sas 4/bouchon 38 est enfoncé à fond dans l'embout borgne 39, la base de l'embout 35 butant contre l'extrémité du goulot 2, le bouchon 38 obture les ouvertures 41 et isole l'intérieur du récipient 1 du sas 4 et de l'extérieur, comme représenté sur la demi-vue de droite de la figure 8.

Par contre, lorsque l'ensemble embout 35 est tiré vers l'extérieur, le bouchon 38 venant buter contre la base du goulot 2, les ouvertures 41 sont dégagées et l'intérieur du récipient communique librement avec le sas 4 et l'embout 36 débouchant à l'extérieur après enlèvement du capuchon 37, comme représenté sur la demi-vue de gauche de la figure 8.

La figure 9 représente une autre variante du conditionnement selon l'invention et de l'obturateur dans lequel un sas 4 est associé à une fermeture par rotation dans le goulot inversé 42 d'un récipient 1. Le sas 4 est limité par un corps 43 fermé par un embout cônique 44 encliqueté terminé par un ajutage axial 45 de distribution, fermé à volonté par un capuchon classique 46. Le bouchon 47 est ajouré et présente un épaulement annulaire interne 48 sur lequel vient porter le bord périphérique d'un élément filtrant amont, dont la face centrale inférieure est ainsi dégagée du fond du bouchon 47.

Un téton axial 49 issu du bouchon 47 est encliqueté en rotation dans une paroi 50 obturant partiellement l'extrémité interne du goulot inversé 42. La paroi 50 et le bouchon 47 du corps 43 présentent des ouvertures identiques 51,52 respectivement, en arcs de cercle de moins de 180 degrés, 90 degrés par exemple, que l'on peut amener à volonté par rotation du corps 43, soit en regard les unes des autres pour faire communiquer l'intérieur du récipient 1 avec le sas 4 et l'extérieur par l'embout 45, après avoir enlevé le capuchon 46, soit en regard, chacune et respectivement, du bouchon 47 du corps 43 et de la paroi 50 du goulot inversé 42, pour isoler l'intérieur du récipient 1 du sas 4 et de l'extérieur, comme représenté sur la figure 9.

Quel que soit le mode de mise en oeuvre des sas 4, il importe impérativement que les assemblages présentent des étanchéités telles qu'il ne puisse y avoir de passage pour le liquide autrement qu'à travers le sas, ce qui peut être réalisé classiquement par des lèvres nervurées, bourrelets ou des joints élastiques.

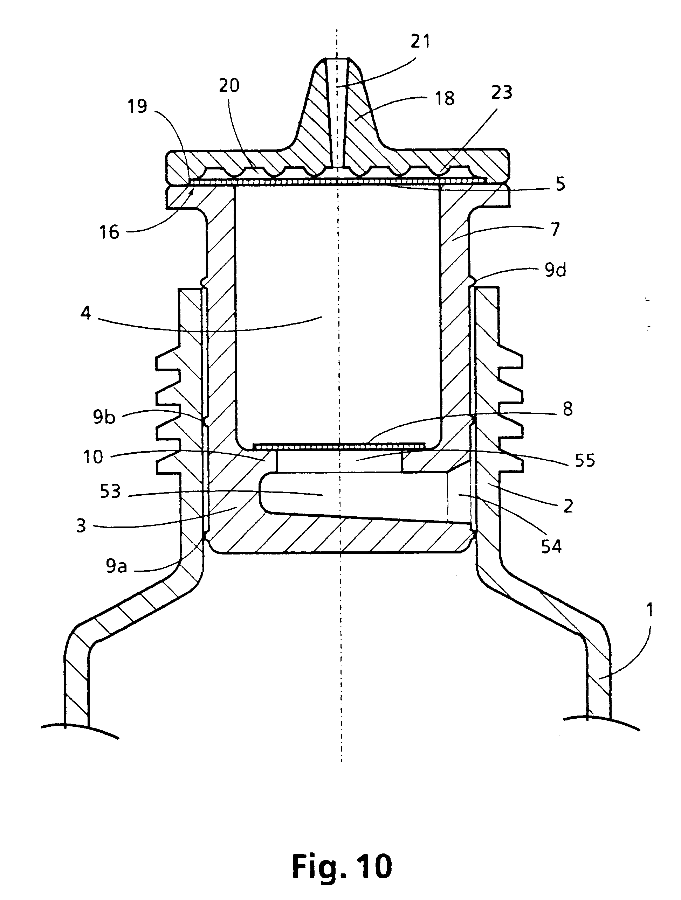

La figure 10 représente une forme de réalisation préférée du conditionnement selon l'invention dans laquelle l'ensemble sas 4/bouchon 3 est constitué d'une seule pièce par un corps tubulaire 7.

Le bouchon 3 constituant l'extrémité du corps 7 appelée à communiquer avec l'intérieur du récipient 1 comporte une chambre 53 de réception d'un produit destiné à être mélangé au liquide contenu dans le récipient 1 lors de la première mise en position d'utilisation du contionnement. Cette chambre 53 débouche par une première ouverture latérale 54 dans la périphérie du corps 7 au niveau du bouchon 3 entre deux nervures annulaires périphériques 9a,9b du corps 7, la nervure 9a étant en appui étanche avec la périphérie intérieure du goulot 2 du récipient 1 dans la position de stockage de l'ensemble sas 4/bouchon 3 et la nervure 9b étant en appui étanche avec la périphérie intérieure du goulot 2, quelle que soit la position de l'ensemble sas 4/bouchon 3.

Une nervure annulaire périphérique supplémentaire 9d du corps 7 est prévue pour parfaire l'étanchéité en position d'utilisation et pour faire en sorte que l'étanchéité soit assurée, quelle que soit la position d'utilisation ou de stockage, par deux nervures.

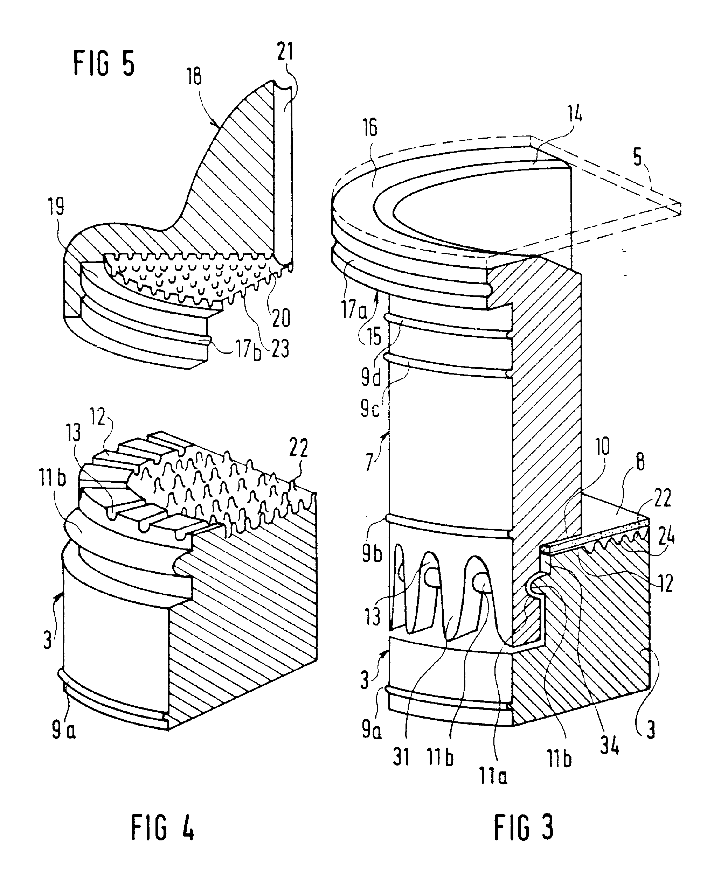

Une seconde ouverture 55 de la chambre 53 débouche dans le sas 4 en regard d'un élément de filtration amont 8. Cette seconde ouverture permet une fois le produit contenu dans la chambre 53 mélangé au liquide, d'assurer la communication entre l'intérieur du récipient 1 et le sas 4 par l'intermédiaire de la chambre 53 jouant alors le rôle des canaux 13 de la forme de réalisation représentée aux figures 1 à 5.

Le conditionnement représenté à la figure 10 comporte également un embout 18 présentant un canal axial 21 de distribution, débouchant d'une part, extérieurement et, d'autre part, au centre d'une face hérissée de villosités 23 ménageant entre elles des canaux 20 intercommuniquants et bordée d'un épaulement annulaire périphérique 19 formant un appui annulaire étanche pour la périphérie d'un élément de filtration aval 5 en regard de la face radiale externe 16 du corps 7.

La séparation entre le sas 4 et le bouchon 3 est constituée par un épaulement 10 d'appui annulaire étanche de l'élément de filtration amont 8 en périphérie et qui ménage l'ouverture 55.

Un capuchon semblable à celui décrit à l'appui des figures 1 à 5, et jouant le même rôle est prévu pour coiffer l'ensemble.

Tous les éléments du conditionnement peuvent être aisément réalisés par moulages de matières plastiques identiques ou différentes, ou bien en matériaux divers usinés.

## Revendications

1. Conditionnement pour liquide à épurer, comprenant un récipient (1) dont un goulot (2) renferme un sas (4) de réception d'au moins un élément de traitement du liquide lors de son expulsion hors du récipient (1), caractérisé en ce que ledit sas (4) est solidaire d'un obturateur ou bouchon mobile (3) déplaçable au moins lors d'une première utilisation entre une position de stockage où il isole l'intérieur du récipient (1) du sas (4) et une position d'utilisation où il permet une communication entre l'intérieur du récipient (1) et l'intérieur du sas (4), ledit obturateur (3) restant solidaire dudit sas (4) qui présente un volume intérieur constant, et ledit sas étant limité par un élément amont (8) de filtration et de retenue permanente dudit élément de traitement dans le sas.

2. Conditionnement, selon la revendication 1, caractérisé en ce que le sas renferme une substance (6) épuratrice insoluble dans le liquide retenue entre ledit élément de filtration amont (8) et un élément de filtration aval (5).

3. Conditionnement, selon la revendication 2, caractérisé en ce que ladite substance épuratrice est propre à éliminer un conservateur contenu dans le liquide à épurer.

4. Conditionnement, selon l'une des revendications 2 ou 3, caractérisé en ce que l'élément de filtration aval (5) est un filtre bactériologique.

5. Conditionnement, selon l'une quelconque des revendications 1 à 4, caractérisé en ce que les éléments de filtration (5,8), notamment l'élément de filtration amont (8), ne se laissent traverser par le liquide à épurer que sous une différence de pression minimum prédéterminée.

6. Conditionnement selon l'une quelconque des revendications 1 à 5, caractérisé en ce qu'il comporte des moyens de variation du volume intérieur du récipient (1).

7. Conditionnement selon la revendication 6, caractérisé en ce que lesdits moyens consistent en des parois élastiquement déformables du récipient (1).

8. Conditionnement, selon la revendication 6, caractérisé en ce que lesdits moyens comportent au moins une paroi mobile du récipient (1) de manière à diminuer son volume au fur et à mesure de l'expulsion du liquide qu'il contient.

9. Conditionnement, selon l'une quelconque des revendications 1 à 8, caractérisé en ce que le sas et le bouchon forment un ensemble unitaire comprenant un corps tubulaire (7) ; le corps comportant au moins un conduit de passage du liquide à travers le bouchon et, un fond qui ferme ces moyens de passage par coopération étanche avec le goulot (2) du récipient (1) au moins avant la première utilisation.

10. Conditionnement, selon l'une quelconque des revendications 1 à 9, caractérisé en ce que l'ensemble sas-bouchon est constitué par un corps tubulaire (7) de révolution présentant à son extrémité appelée à communiquer avec l'extérieur, un évasement interne (14) dans une collerette terminale (15) dont la face radiale externe forme un appui annulaire étanche (16) pour la périphérie dudit élément de filtration aval (5).

11. Conditionnement, selon la revendication 10, caractérisé en ce qu'il comporte un embout distributeur (18) présentant un épaulement annulaire interne (19) appliquant la périphérie de l'élément de filtration aval (5) contre l'épaulement d'appui annulaire (16) de la collerette (15) et présentant des moyens de passage (20) du liquide entre la face externe de l'élément de filtration aval (5) et un canal axial (21) de distribution de l'embout (18).

12. Conditionnement, selon la revendication 10 ou 11, caractérisé en ce que l'ensemble sas-bouchon (4,3) a une longueur supérieure à celle du goulot (2) du récipient (1) dans lequel il coulisse, le bouchon (3) et le tronçon du corps tubulaire (7) de même diamètre présentant des lèvres ou bourrelets annulaires d'étanchéité (9) dans le goulot (2) en position de stockage (9a,9c) et en position d'utilisation (9b,9d) ; la collerette (15) formant butée sur le bord (25) du goulot du récipient (1) pour limiter l'enfoncement de l'ensemble unitaire sas-bouchon (4,3) vers la position d'utilisation où au moins une ouverture latérale (13) du bouchon est dégagée du goulot (2) alors qu'elle est enfermée et isolée dans le goulot (2) en position de stockage.

13. Conditionnement, selon la revendication 11 ou 12, caractérisé en ce qu'il comprend un capuchon (26) vissant à l'extérieur du goulot (2) et comportant un téton axial (27) d'obturation du canal de distribution (21) de l'embout (18) ainsi qu'un prolongement annulaire interne (28) d'appui contre la face externe de l'embout (18) pour enfoncer manuellement celui-ci dans le goulot (2) vers la position d'utilisation, le capuchon (26) comportant une bague frangible (29) de sécurité prolongeant sa jupe (30) et portant contre le col (33) du récipient (1) de sorte qu'après la première utilisation, le capuchon (26) et l'embout (18) deviennent inopérant à provoquer le déplacement de l'ensemble sas (4)/bouchon (3).

14. Conditionnement, selon l'une quelconque des revendications 9 à 13, caractérisé en ce que l'ensemble sas (4)/bouchon (3) est constitué d'une seule pièce par ledit corps tubulaire (7) et en ce que ce dernier comporte, dans son extrémité appelée à communiquer avec l'intérieur du récipient (1), une chambre (53) de réception d'un produit destiné à être mélangé au liquide lors d'une mise en position d'utilisation et débouchant par une première ouverture latérale (54) dans la périphérie dudit bouchon (3) et par une seconde ouverture (55) dans ledit sas (4) en regard dudit élément de filtration amont (8).

15. Conditionnement, selon la revendication 14, caractérisé en ce que ledit corps tubulaire (7) comporte un épaulement d'appui annulaire étanche (10) dudit élément de filtration amont (8) en périphérie qui constitue la séparation entre le sas (4) et le bouchon (3).

16. Conditionnement, selon l'une des revendications 14 ou 15, caractérisé en ce que ladite chambre (53) de réception est dans la position de stockage, isolée de l'intérieur du récipient (1), et en communication avec ce dernier dans la position d'utilisation, ledit produit se trouvant, lors d'une première mise en position d'utilisation, déversé dans ledit récipient (1).

17. Conditionnement, selon l'une quelconque des revendications 9 à 13, caractérisé en ce que ledit corps tubulaire (7) comporte des moyens d'encliquetage étanches (11) pour un bouchon (3) de même diamètre extérieur que le corps tubulaire (7) dont un bord périphérique (12) applique ledit élément de filtration amont (8) contre l'épaulement d'appui (10) du corps tubulaire.

18. Conditionnement selon la revendication 17, caractérisé en ce que le bouchon comporte des canaux radiaux (13) pour le libre passage du liquide, débouchant en dessous de l'élément de filtration amont (8), côté récipient (1).

19. Conditionnement, selon l'une quelconque des revendications 17 ou 18, caractérisé en ce que ledit corps tubulaire présente à son extrémité appelée à communiquer avec l'intérieur du récipient (1) un épaulement interne d'appui annulaire étanche (10) dudit élément de filtration amont (8) en périphérie.

20. Conditionnement, selon la revendication 11 et l'une quelconque des revendications 11 et 17 à 19, caractérisé en ce que les faces du bouchon (3) et de l'embout (18) en regard des éléments de filtration (5,8), hormis la périphérie, présentent des aspérités d'appui (22,23) pour les faces des éléments de filtration, qui ménagent entre elles des canaux intercommuniquants (20,24) de circulation du liquide.

21. Conditionnement, selon l'une quelconque des revendications 11 à 20, caractérisé en ce que l'ensemble sas (4) obturateur (3) est déplaçable à volonté entre ladite position de stockage et ladite position d'utilisation.

22. Conditionnement selon l'une quelconque des revendications 11 à 21, caractérisé en ce que l'embout (35) comporte des moyens de préhension permettant à un utilisateur d'imprimer un mouvement de translation à l'ensemble sas (4)/bouchon (38).

23. Conditionnement selon la revendication 21, caractérisé en ce que l'ensemble sas (4)/bouchon (47) est associé à une fermeture par rotation dans un goulot inversé (42) du récipient (1), ledit bouchon (47) présentant un fond ajouré et un téton axial (49) encliqueté en rotation dans une paroi (50) obturant partiellement l'extrémité interne du goulot inversé (42).

24. Conditionnement selon l'une quelconque des revendications 2 à 23, caractérisé en ce que ledit élément de filtration aval est sélectivement perméable à un premier constituant du liquide à expulser et en ce que ledit élément de filtration amont est perméable à tout, le liquide, ou sélectivement perméable au constituant à expulser et à un second constituant à piéger dans le sas lors de l'expulsion.

## Patentansprüche

1. Behälter für eine zur Reinigung bestimmte Flüssigkeit, bestehend aus einer Flasche (1), deren Hals (2) eine Aufnahmeschleuse (4) enthält, die wenigstens ein Mittel
zur Behandlung der Flüssigkeit beim Austritt aus der Flasche (1) enthält, dadurch gekennzeichnet, daß die Schleuse (4) mit einer Deckelvorrichtung oder einem beweglichen Deckel (3) versehen ist, der sich frühestens bei der ersten Ingebrauchnahme aus einer Lagerposition, in der er die Flasche (1) von der Schleuse (4) trennt, in eine Gebrauchsposition verlagert, in der ein Durchgang zwischen dem Inneren der Flasche (1) und dem Inneren der Schleuse (4) entsteht, wobei die Deckelvorrichtung (3) mit der Schleuse (4), die ein gleichbleibendes Fassungsvermögen hat, verbunden bleibt, und die Schleuse ihrerseits durch eine vorgelagerte Filter- und Rückhaltevorrichtung (8) geschlossen ist.

2. Behälter gemäß Patentanspruch 1, dadurch gekennzeichnet, daß die Schleuse eine in der Flüssigkeit unlösliche Reinigungssubstanz (6) enthält, die zwischen der unteren Filtervorrichtung (8) und einer oberen (5) eingeschlossen ist.

3. Behälter gemäß Patentanspruch 2, dadurch gekennzeichnet, daß die Reinigungssubstanz dazu geeignet ist, ein in der Flüssigkeit enthaltenes Konservierungsmittel abzuscheiden.

4. Behälter gemäß Patentanspruch 2 oder 3, dadurch gekennzeichnet, daß die obere Filtervorrichtung (5) in einem bakteriologischen Filter besteht.

5. Behälter gemäß einem beliebigen der Patentansprüche 1 bis 4, dadurch gekennzeichnet, daß die Filtervorrichtungen (5,8), insbesondere die untere Filtervorrichtung (8) die zur Reinigung vorgesehene Flüssigkeit nur durch Ausübung eines zuvor festgesetzten Mindestdruckeinflusses eintreten läßt.

6. Behälter gemäß einem beliebigen der Patentansprüche 1 bis 5, dadurch gekennzeichnet, daß er Vorrichtungen zur Änderung des Innenvolumens der Flasche (1) enthält.

7. Behälter gemäß Patentanspruch 6, dadurch gekennzeichnet, daß diese Vorrichtungen in elastisch verformbaren Wandungen der Flasche (1) bestehen.

8. Behälter gemäß Patentanspruch 6, dadurch gekennzeichnet, daß diese Vorrichtungen in mindestens einer beweglichen Wandung der Flasche bestehen, mit der das Volumen mit der allmählichen Ausschüttung der enthaltenen Flüssigkeit fortlaufend verringert wird.

9. Behälter gemäß einem beliebigen der Patentansprüche 1 bis 8, dadurch gekennzeichnet, daß die Schleuse und der Deckel eine Einheit bilden, die einen Hohlkörper (7) enthält, wobei dieser Hohlkörper mindestens einen Durchlaßkanal für die Flüssigkeit durch den Deckel sowie einen Boden aufweist, der diese Durchlaßkanäle durch Abdichtung des Flaschenhalses (2) vor dem ersten Gebrauch abschließt.

10. Behälter gemäß einem beliebigen der Patentansprüche 1 bis 9, dadurch gekennzeichnet, daß die Einheit Schleuse/Deckel aus einem umgekehrten Hohlkörper (7) besteht, der an seinem nach außen gerichteten Ende eine Querschnittserweiterung (14) inmitten eines Kragens (15) aufweist, dessen äußere Rundfläche eine abgedichtete ringförmige Auflage (16) für den Rand der oben beschriebenen obere Filtervorrichtung (5) bildet.

11. Behälter gemäß Patentanspruch 10, dadurch gekennzeichnet, daß er eine Ausschüttvorrichtung (18) mit einen ringförmigen Rundvorsatz aufweist, der die Einfassung der oberen Filtervorrichtung (5) gegen die dichte ringförmige Auflage (16) des Kragens (15) drückt und Durchlaßkanäle (20) zwischen der Außenfläche der oberen Filtervorrichtung (5) und dem Durchflußkanal (21) der Ausschüttvorrichtung (18) aufweist.

12. Behälter gemäß Patentanspruch 10 oder 11, dadurch gekennzeichnet, daß die Einheit Schleuse/Deckel (4,3) länger ist als der Flaschenhals (2), in dem sie sich bewegt, wobei der Deckel (3) und das Hohlkörperende (7) gleichen Durchmessers in dem Flaschenhals (2) in Lagerstellung (9a, 9c) und in Gebrauchsstellung (9b, 9d) ringförmige Dichtungslippen oder -wülste (9) aufweist; der Kragen (15) bildet am Außenrand (25) des Flaschenhalses eine Sperre, um das Eindringen der Einheit Schleuse/Deckel (4. 3) in die Gebrauchsstellung zu verhindern, wobei wenigstens eine seitliche Öffnung (13) des Deckels vom Flaschenhals (2) liegt frei, während sei in dem Flaschenhals (2) in Lagerstellung eingeschlossen und isoliert ist.

13. Behälter gemäß Patentanspruch 11 oder 12, dadurch gekennzeichnet, daß er am äußeren Flaschenhals (2) eine verschraubbare Verschlußkappe (26) sowie einen senkrechten Dorn (27) zum Deckel des Durchflußkanals (21) der Ausschüttvorrichtung (18) aufweist , ferner eine innere ringförmige Verlängerung (28), die einen Druck gegen die Außenseite der Ausschüttvorrichtung (18) ausübt, mit dem sie manuell in den Flaschenhals (2) geschoben und so in Gebrauchsstellung verbracht wird, wobei die Verschlußkappe (26) einen Sicherungsring (29) aufweist, der die Kappenwandung (30) zum Flaschenkolben (33) hin verlängert, sodaß nach dem ersten Gebrauch die Verschlußkappe (26) und die Ausschüttvorrichtung (18) eine Verlagerung der Einheit Schleuse/Deckel (4/3) nicht mehr erlauben.

14. Behälter gemäß Patentanspruch 9 bis 13, dadurch gekennzeichnet, daß die Einheit Schleuse/Deckel (4/3) aus einem Stück in Form des Hohlkörpers (7) besteht und daß der Deckel an dem Ende, das mit dem Inneren der Flasche (1) in Verbindung steht, eine Auffangkammer (53) für ein Produkt aufweist, das zur Beimischung zu der Flüssigkeit bestimmt ist, sobald die Flasche in Gebrauchstellung gebracht wird, und die durch eine erste seitliche Öffnung (54) in die Umgebung des Deckels (3) und durch eine zweite Öffnung (55) gegenüber der unteren Filtervorrichtung in die Schleuse (4) mündet.

15. Behälter gemäß Patentanspruch 14, dadurch gekennzeichnet, daß der Hohlkörper (7) an der unteren Filtervorrichtung (8) einen umlaufenden ringförmigen dichten Vorsprung (10) aufweist, der die Trennung zwischen der Schleuse (4) und dem Deckel (3) bewirkt.

16. Behälter gemäß einem der Patentansprüche 14 oder 15, dadurch gekennzeichnet, daß die Auffangkammer (53) in der Lagerstellung vom Flascheninneren getrennt und in Gebrauchsstellung damit verbunden ist, wobei das Produkt bei der erstmaligen Versetzung in Gebrauchsstellung in die Flasche (1) ausgeschüttet wird.

17. Behälter gemäß einem beliebigen der Patentansprüche 9 bis 13, dadurch gekennzeichnet, daß der Hohlkörper (7) mit dichten Haltevorrichtungen (11) für einen Deckel (3) versehen ist, der denselben Außendurchmesser wie der Hohlkörper (7) hat, dessen Außenrand (12) die untere Filtervorrichtung (8) gegen den ringförmigen Vorsprung (10) des Hohlkörpers preßt.

18. Behälter gemäß Patentanspruch 17, dadurch gekennzeichnet, daß der Deckel strahlenförmige Rinnen (13) zum freien Abfluß der Flüssigkeit enthält, die sich unterhalb der unteren Filtervorrichtung (8) seitlich der Flasche (1) befinden.

19. Behälter gemäß Patentanspruch 17 oder 18 , dadurch gekennzeichnet, daß der Hohlkörper an seinem mit dem Flascheninneren verbundenen Ende einen inneren ringförmigen dichten Vorsprung (10) aufweist, der die untere Filtervorrichtung umgibt.

20. Behälter gemäß Patentanspruch 11 bzw. einem beliebigen der Patentansprüche 11 und 17 bis 19, dadurch gekennzeichnet, daß die Seiten des Deckels (3) und der Ausschüttvorrichtung (18), die den Filtervorrichtungen (5,8) zugewandt sind, außer der Peripherie unebene Auflagen (22,23) für die Flächen der Filtervorrichtungen bieten, die miteinander durch Verbindungskanäle (20,24) für den Durchlaß der Flüssigkeit verbunden sind.

21. Behälter gemäß einem beliebigen der Patentansprüche 11 bis 20, dadurch gekennzeichnet, daß die Einheit Schleuse/Deckel (4/3) beliebig zwischen der Lager- und der Gebrauchsstellung verstellbar ist.

22. Behälter gemäß einem beliebigen der Patentansprüche 11 bis 21, dadurch gekennzeichnet, daß der kegelförmige Ansatz Griffvorrichtungen aufweist, die dem Verbraucher erlauben, die Einheit Schleuse/Deckel (38) in Durchlaßstellung zu bringen.

23. Behälter gemäß Patentanspruch 21, dadurch gekennzeichnet, daß die Einheit Schleuse(4)/Deckel (47) mit einem Drehdeckel in einem umgekehrten Flaschenhals (42) versehen ist, wobei der Deckel (47) einen durchlässigen Boden und einen Dorn (49) hat, der durch Drehung in eine Wandung (50) einrastet und das innere Ende des umgedrehten Flaschenhalses (42) teilweise verschließt.

24. Behälter gemäß einem beliebigen der Patentansprüche 2 bis 23, dadurch gekennzeichnet, daß die obere Filtervorrichtung wahlweise für einen ersten zur Ausschüttung gelangenden Bestandteil der Flüssigkeit durchlässig ist, ferner dadurch, daß diese obere Filtervorrichtung für die gesamte Flüssigkeit oder für deren zur Verwendung bestimmten Bestandteil samt einen Zusatz, der sich vor der Ausschüttung in der Schleuse befindet, durchlässig ist.

## Claims

1. A package for a liquid to be purified, comprising a container (1) having a neck (2) which surrounds a lock-chamber (4) for receiving at least one element for the treatment of the liquid when it is discharged from the container (1), characterized in that said lock-chamber (4) is rigidly fixed to an obturator or movable plug (3) which is capable of displacement at least at the time of initial use between a storage position in which it isolates the interior of the container (1) from the lock-chamber (4) and a position of use in which it establishes a communication between the interior of the container (1) and the interior of the lock-chamber (4), said obturator (3) being intended to remain rigidly fixed to said lock-chamber (4) which has a constant internal volume, said lock-chamber being limited by an upstream filter element (8) which continuously keeps the said element for treatment in the lock-chamber.

2. A package in accordance with claim 1, characterized in that the lock-chamber contains a purifying substance (6) which is insoluble in the liquid retained between an upstream filter element (8) and a dowstream filter element (5).

3. A package in accordance with claim 2 characterized in that said purifying substance is capable of removing a preservative contained in the liquid to be purified.

4. A package in accordance with claim 2 or claim 3, characterized in that the downstream filter element (5) is a bacteriological filter.

5. A package in accordance with any one of claims 1 to 4, characterized in that the filter elements (5, 8) and especially the upstream filter element (8) allow the liquid to be purified to pass through only with a predetermined minimum pressure difference.

6. A package in accordance with any one of claims 1 to 5, characterized in that said package comprises means for varying the internal volume of the container (1).

7. A package in accordance with claim 6, characterized in that the means aforesaid consist of elastically deformable walls of the container (1).

8. A package in accordance with claim 6, characterized in that the means aforesaid comprise at least one movable wall of the container (1) so as to reduce its volume as the liquid contained therein is being discharged.

9. A package in accordance with any one of claims 1 to 8, characterized in that the lock-chamber and the plug form a unit assembly having a tubular body (7), the body being provided with at least one duct for the flow of liquid through the plug and an end-wall which closes said flow means by fluid-tight cooperation with a neck (2) of the container (1) at least prior to initial use.

10. A package in accordance with any one of claims 1 to 9, characterized in that the lock-chamber/ plug unit is constituted by a tubular body (7) of revolution, the end of said body which is intended to communicate with the exterior being provided with an internal flared-out portion (14) in a terminal annular flange (15), the external radial face of which forms a fluid-tight annular support (16) for the periphery of said downstream filter element (5).

11. A package in accordance with claim 10, characterized in that said package comprises a delivery nozzle (18) having an internal annular shoulder (19) which applies the periphery of the downstream filter element (5) against the annular bearing shoulder (16) of the annular flange (15) and having means (20) for causing the liquid to flow between the external face of the downstream filter element (5) and an axial delivery duct (21) of the nozzle (18).

12. A package in accordance with claim 10 or claim 11, characterized in that the length of the lock-chamber/plug unit (4, 3) is greater than that of the container (1) neck (2) in which the unit slides, the plug (3) and the section of the tubular body (7) which has the same diameter being provided with annular sealing lips or beads (9) in the neck (2) in the storage position (9a, 9c) and in the position of use (9b, 9d), the annular flange (15) being intended to form a stop on the edge (25) of the container (1) neck in order to limit the extent of downward travel of the lock-chamber/plug unit (4, 3) towards the position of use in which at least one lateral opening (13) of the plug is freed from the neck (2) whereas it is enclosed and isolated within the neck (2) in the storage position.

13. A package in accordance with claim 11 or claim 12, characterized in that said package comprises a cap (26) which is screwed onto the exterior of the neck (2) and which has an axial stud (27) for shutting-off the delivery duct (21) of the nozzle (18) as well as an internal annular extension (28) applied against the external face of the nozzle (18) in order to thrust said nozzle by hand within the neck (2) in the downward direction towards the position of use, the cap (26) being provided with a breakable safety ring (29) forming an extension of its skirt (30) and applied against the neck base (33) of the container (1) so that after initial use, the cap (26) and the nozzle (18) are no longer capable of causing the displacement of the unit consisting of lock-chamber (4) and plug (3).

14. A package in accordance with any one of claims 9 to 13, characterized in that the unit consisting of lock-chamber (4) and plug (3) is formed in a single piece by said tubular body (7) and that the end of said body which is intended to communicate with the interior of the container (1) is provided with a chamber (53) which receives a product to be mixed with the liquid at the time of initial setting in the position of use and which discharges through a first lateral opening (54) into the periphery of said plug (3) and through a second opening (55) into said lock-chamber (4) opposite to said upstream filter element (8).

15. A package in accordance with claim 14, characterized in that said tubular body (7) has a fluid-tight annular shoulder (10) for supporting said upstream filter element (8) at the periphery which constitutes the separation between the lock-chamber (4) and the plug (3).

16. A package in accordance with claim 14 or claim 15, characterized in that said receiving chamber (53) is isolated from the interior of the container (1) in the storage position and in communication with said container in the position of use, said product being poured into said container (1) at the time of initial setting in the position of use.

17. A package in accordance with any one of claims 9 to 13, characterized in that said tubular body (7) comprises fluid-tight snap-engagement means (11) for a plug (3) having the same external diameter as the tubular body (7), a peripheral edge (12) of which applies said upstream filter element (8) against the annular bearing shoulder (10) of the tubular body.

18. A package in accordance with claim 17, characterized in that the plug has radial channels (13) for the free flow of liquid, said channels being intended to open beneath the upstream filter element (8) on the side nearest the container (1).

19. A package in accordance with claim 17 or claim 18, characterized in that said tubular body is provided at the end which is intended to communicate with the interior of the container (1) with a fluid-tight internal annular shoulder (10) for supporting said upstream filter element (8) at the periphery.

20. A package in accordance with claim 11 and any one of claims 11 and 17 to 19, characterized in that the faces of the plug (3) and of the nozzle (18) opposite to the filter elements (5, 8), apart from the periphery, have rough surface excrescences (22, 23) which support the faces of the filter elements and form between them intercommunicating channels (20, 24) for the circulation of liquid.

21. A package in accordance with any one of claims 11 to 20 characterized in that the unit consisting of lock-chamber (4) and obturator (3) is displaceable at will between said storage position and said position of use.

22. A package in accordance with any one of claims 11 to 21 characterized in that the end-piece (35) has means for gripping by the user and for imparting a movement of translation to the unit consisting of lock-chamber (4) and plug (38).

23. A package in accordance with claim 21, characterized in that the unit consisting of lock-chamber (4) and plug (47) is associated with a rotational closure device within an inverted neck (42) of the container (1), said plug (47) being provided with a bottom end-wall with open portions and an axial stud (49) latched in rotation in a wall (50) which partially shuts-off the internal end of the inverted neck (42).

24. A package in accordance with any one of claims 2 to 23, characterized in that said downstream filter element is selectively permeable to a first constituent of the liquid to be discharged and that said upstream filter element is permeable to the entire quantity of liquid or selectively permeable to the constituent to be discharged and to a second constituent to be trapped in the lock-chamber at the time of discharge.
